# EUROPEAN PATENT SPECIFICATION

(11) **EP 3 173 785 B1**
(45) Date of publication and mention of the grant of the patent: **25.11.2020**
(21) Application number: 15823991.3
(22) Date of filing: 21.07.2015
(51) Int. Cl.: G01N 33/48, A61K 49/00, C07D 401/14

(54) **LABELING REAGENT CONTAINING A MOLECULARLY TARGETED DRUG**
MARKIERUNGSREAGENS MIT EINEM MOLEKULAR ABGEZIELTEN ARZNEIMITTEL
RÉACTIF DE MARQUAGE CONTENANT UN MÉDICAMENT À CIBLE MOLÉCULAIRE

(30) Priority: 23.07.2014 JP 2014149568
(43) Date of publication of application: 31.05.2017
(73) Proprietor: Konica Minolta, Inc., Tokyo 100-7015 (JP)
(72) Inventor: AIMIYA, Takuji, Tokyo 100-7015 (JP); FURUSAWA, Naoko, Tokyo 100-7015 (JP)
(74) Representative: Henkel & Partner mbB
(86) International application number: PCT/JP2015/070676
(87) International publication number: WO 2016/013541

(56) References cited:
- WO-A1-2012/133047
- JP-A- 2001 318 097
- JP-A- 2002 508 507
- JP-A- 2005 514 430
- JP-A- 2007 521 338
- JP-A- 2013 502 580
- JP-A- 2013 518 581
- US-A1- 2005 153 371
- SHUKLA SUNEET ET AL.: 'Synthesis and characterization of a BODIPY conjugate of the BCR-ABL kinase inhibitor Tasigna (nilotinib): evidence for transport of Tasigna and its fluorescent derivative by ABC drug transporters' MOLECULAR PHARMACEUTICS vol. 8, no. 4, 01 January 2011, pages 1292 - 1302, XP055387540 DOI: 10.1021/MP2001022
- ZHOU, QIANGHUI ET AL.: 'Bioconjugation by Native Chemical Tagging of C-H Bonds' JOURNAL OF THE AMERICAN CHEMICAL SOCIETY vol. 135, no. 35, 04 September 2013, pages 12994 - 12997, XP055337810 DOI: 10.1021/JA407739Y
- EL-MASHTOLY SAMIR F ET AL.: 'Label-free imaging of drug distribution and metabolism in colon cancer cells by Raman microscopy' ANALYST vol. 139, 01 January 2014, pages 1155 - 1161, XP055387544 DOI: 10.1039/C3AN01993D
- JERRY C. CHANG ET AL.: 'A Fluorescence Displacement Assay for Antidepressant Drug Discovery Based on Ligand-Conjugated Quantum Dots' JOURNAL OF THE AMERICAN CHEMICAL SOCIETY vol. 133, no. 44, 01 January 2011, pages 17528 - 17531, XP055387555 DOI: 10.1021/JA204301G

## Description

### TECHNICAL FIELD

The present invention relates to a labeling agent which can be used for, for example, measuring the amount of a molecular target drug binding to a specific biological substance (target molecule) in a tissue. More particularly, the present invention relates to a labeling agent comprising: axitinib as the molecular target drug; and a fluorescent substance-integrated nanoparticle as a label bound thereto.

### BACKGROUND ART

Molecular target drugs are pharmaceuticals that treat a specific disease by elucidating a gene and/or protein that is the cause of the disease and acting on a biological substance involved in the abnormal expression, signal transduction and the like of such a gene and protein at the molecular level. In recent years, several low-molecular-weight compounds and antibodies have been put into practical use as molecular target drugs against cancers, autoimmune diseases and the like.

In the discovery of such molecular target drugs, it is important to measure the amount of a drug of interest binding to a specific biological substance (target molecule) because it leads to screening and the like of candidate drugs. For this purpose, a method in which a candidate drug coupled with a label, such as a radioisotope, an enzyme that develops a color upon reaction with a specific substrate, or a phosphor, is allowed to bind with a target molecule and the amount of the thus bound candidate drug is measured based on a signal originating from the label is employed.

For example, Patent Document 1 (WO 2012/133047 A1) describes an immunohistological staining method in which an antibody (e.g. trastuzumab) used in an antibody preparation is labeled and allowed to bind to an antigen (e.g. HER2) targeted by the antibody preparation, and it is also described therein, for example, that quantum dots and fluorescent substance-integrated nanoparticles (e.g. organic fluorescent dye-integrated silica nanoparticles) can be used as a label for labeling the antibody and that the immunohistological staining method can be applied as a method of assessing the effectiveness of the antibody preparation. The use of a fluorescent substance-integrated nanoparticle as a label for immunostaining enables to perform highly accurate quantification and is thus more preferred than the use of a quantum dot or a color developer based on a conventional enzyme.

Meanwhile,low-molecular-weight compound have also been used as well as antibodies conventionally as molecular target drugs for various diseases, and they are utilized not only as active ingredients of therapeutic pharmaceuticals but also in the preparation of a conjugate for diagnosis that is used in an assay to detect a target molecule in a sample. For example, Patent Document 2 (Japanese Translated PCT Patent Application Laid-open No. 2007-521338) describes compounds having a structure in which components useful in biopharmaceutical or bioassay applications, such as a binder, a labeling compound (e.g. an organic fluorescent dye) and a therapeutic agent (e.g. a kinase inhibitor), are bound to the terminals of a prescribed divalent linker molecule and, compounds having a nitrogen atom-containing aromatic ring (hereinafter, referred to as "compounds comprising a nitrogen-containing aromatic ring") such as sorafenib (see Compound No. VI shown in the table of the paragraph [0070]) are mentioned therein as the kinase inhibitor.

However, although Patent Document 2 discloses embodiments where, as a site of a compound having a nitrogen atom-containing aromatic ring (nitrogen-containing aromatic ring) to be bound to a linker molecule, (i) when the nitrogen-containing aromatic ring is positioned at a terminal of the compound, the nitrogen atom of the nitrogen-containing aromatic ring or the nitrogen atom of a nitrogen atom-containing functional group (e.g. an amino group, an amide group or a carbamoyl group) directly or indirectly bound to the nitrogen-containing aromatic ring is selected or (ii) when a heteroatom-free aromatic ring is positioned at a terminal of the compound, a carbon atom of the aromatic ring is selected, there is no disclosure with regard to (iii) selection of a carbon atom of the nitrogen-containing aromatic ring when the nitrogen-containing aromatic ring is positioned at a terminal of the compound nor the technical significance thereof. For instance, with regard to sorafenib, a general description merely suggests that a divalent linker molecule is bound to the nitrogen atom of a carbamoyl group bound to a nitrogen-containing aromatic ring (pyridyl group) at a terminal, and such an embodiment where a divalent linker molecule is bound to sorafenib as described above is not concretely disclosed in Examples and the like.

Patent Document 3 (Japanese Translated PCT Patent Application Laid-open No. 2012-533579) describes, as an invention aimed at targeting of kidney, a conjugate which comprises a compound(s) carrying carboxyl groups and a prescribed oligomer constituted by *ε*-lysine monomer units and to which an active compound is optionally further covalently bonded, and protein kinase inhibitors such as sorafenib are mentioned as the active compound. For instance, in Example 8 ([0181] to [0189]), it is disclosed that a conjugate (DOTA-*ε*-polylysine-sorafenib derivative) comprising DOTA as the compounds carrying carboxyl groups, *ε*-polylysine, and a sorafenib derivative as the active compound was produced. In this conjugate obtained by the production method of Example 8, *ε*-polylysine was bound to a hydroxyethyl group introduced to a terminal of the sorafenib derivative (a hydroxyethyl group bound to the nitrogen atom of a carbamoyl group bound to a pyridyl group).

Non-Patent Document 1 discloses a general C-H functionalization method for tagging natural products and pharmaceuticals. The functionalization method makes use of an azide-containing sulfonate reagent that allows appendage of an azidoalkyl chain onto a heteraromatic compound, the product of which can subsequently be attached to a monoclonal antibody by a "click" reaction so as to produce a drug-antibody conjugate.

### RELATED ART DOCUMENTS

Patent Document 1: WO 2012/133047 A1
Patent Document 2: Japanese Translated PCT Patent Application Laid-open No. 2007-521338
Patent Document 3: Japanese Translated PCT Patent Application Laid-open No. 2012-533579
Non-Patent Document 1: Zhou et al., Journal of the American Chemical Society 135 (2013), 12994-12997

### SUMMARY OF THE INVENTION

### PROBLEMS TO BE SOLVED BY THE INVENTION

Molecular target drugs composed of a low-molecular-weight compound include a number of compounds that comprise a nitrogen atom-containing aromatic ring, such as axitinib for renal cell carcinoma. However, in bioassays relating to such a compound comprising a nitrogen-containing aromatic ring, when a labeling agent obtained by a conventional production method in which a label is bound via a nitrogen atom of the nitrogen-containing aromatic ring or a nitrogen atom of a nitrogen atom-containing functional group directly or indirectly bound to the nitrogen-containing aromatic ring is used, there is a problem that the signal originating from the label is weak. Particularly, when a fluorescent substance-integrated nanoparticle is used as the label, since the fluorescence intensity representing its binding to a target molecule is weak or the number of bright spots is small, it is difficult to evaluate the binding properties of a molecular target drug.

An object of the present invention is to provide a labeling agent which exhibits excellent binding properties to a target molecule in a bioassay or histological staining method that relates to a compound comprising a nitrogen-containing aromatic ring.

### TECHNICAL SOLUTION

The present inventors discovered that the use of a labeling agent which is different from conventional ones in that a label is bound via a carbon atom of a nitrogen-containing aromatic ring of a compound comprising the nitrogen-containing aromatic ring yields a stronger signal originating from the label, particularly a stronger fluorescence intensity that represents the binding to a target molecule, as well as a greater number of bright spots, thereby completing the present invention. The invention is defined by the claims.

In a first aspect, the present invention thus provides a labeling agent as defined in claim 1. The labeling agent has a structure in which a molecular target drug, which is a compound comprising a nitrogen atom-containing aromatic ring, is bound with a label via a divalent linking group, wherein one end of the divalent linking group is bound to a carbon atom of the nitrogen atom-containing aromatic ring, the molecular target drug is axitinib, and the label is a fluorescent substance-integrated nanoparticle.

In a second aspect, the present invention provides a bioassay as defined in claim 3. The bioassay uses the labeling agent of the first aspect.

In a third aspect, the present invention provides a histological staining method as defined in claim 4. The histological staining method uses the labeling agent of the first aspect.

### ADVANTAGEOUS EFFECTS OF INVENTION

In the labeling agent of the present invention, as compared to conventional labeling agents, the binding of axitinib, which is utilized as a molecular target drug, to a target molecule is improved; therefore, the labeling agent of the present invention enables to more accurately evaluate the effects of the molecular target drug and to more accurately quantify the target molecule existing in a tissue section (pathological specimen) derived from a patient and thereby more accurately estimate the effects of the molecular target drug.

### MODE FOR CARRYING OUT THE INVENTION

### -Immunostaining Agent-

The labeling agent of the present invention is a labeling agent having a structure in which a molecular target drug, namely axitinib as a compound comprising a nitrogen-containing aromatic ring, is bound to a label via a divalent linking group, wherein one end of the divalent linking group is bound to a carbon atom of the nitrogen atom-containing aromatic ring.

### (Molecular Target Drug)

The molecular target drug used in the present invention is a compound comprising a nitrogen-containing aromatic ring, namely axitinib (see the formula below) . In the present invention, the term "compound comprising a nitrogen-containing aromatic ring" thus refers to a compound that belongs to low-molecular-weight preparations, and not antibody preparations, and the "nitrogen-containing aromatic ring" is pyridine as an example of a 6-membered monocyclic compound, or is indazole as an example of a bicyclic compound having a 5-membered ring and a 6-membered ring.

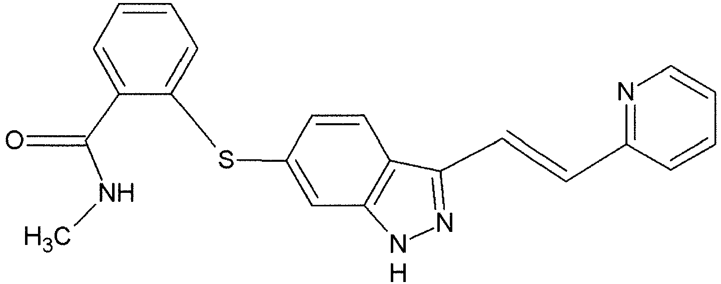

### (Label)

The label used in the present invention is a phosphor that has excellent quantitative properties and allows the binding of the molecular target drug to its target molecule to be accurately evaluated because the use of such a phosphor enables to measure the fluorescence intensity or the number of bright spots as a signal.

More specifically, the phosphor is provided in the form of fluorescent substance-integrated nanoparticles, particularly fluorescent dye-containing resin particles, which are capable of emitting fluorescence at an intensity sufficient for indicating each target molecule as a bright spot and can be detected even by a relatively inexpensive low-sensitivity camera.

The term "phosphor" used herein refers to a substance whose electrons are excited when the substance is irradiated with an electromagnetic wave of a prescribed wavelength (X-ray, UV radiation or visible light) and absorbs the energy thereof and which releases an excess energy during the transition from an excited state to the ground state in the form of an electromagnetic wave, namely "fluorescence", and can be bound with a "probe". Further, the term "fluorescence" has a broad meaning and encompasses phosphorescence that has a long emission lifetime sustaining the emission even after the irradiation with an electromagnetic wave for excitation is terminated; and fluorescence in a narrow sense that has a short emission lifetime.

### • Inorganic Semiconductor Nanoparticle

Examples of inorganic semiconductor nanoparticles suitable for use in the present invention include those which contain a Group II-VI compound, a Group III-V compound or a Group IV element, such as nanoparticles of CdSe, CdS, CdTe, ZnSe, ZnS, ZnTe, InP, InN, InAs, InGaP, GaP, GaAs, Si and Ge. Further, core/shell-type inorganic semiconductor nanoparticles in which any of the above-described inorganic semiconductor nanoparticles is used as a core and a shell is formed outside thereof, such as CdSe/ZnS, CdS/ZnS, InP/ZnS, InGaP/ZnS, Si/SiO₂, Si/ZnS, Ge/GeO₂ and Ge/ZnS, can also be used.

### • Organic Fluorescent Dye

Examples of organic fluorescent dyes suitable for use in the present invention include rhodamine-based dye molecules, squarylium-based dye molecules, cyanine-based dye molecules, aromatic ring-containing dye molecules, oxazine-based dye molecules, carbopyronine-based dye molecules and pyrromethene-based dye molecules. Alternatively, Alexa Fluor (registered trademark, manufactured by Invitrogen)-based dye molecules, BODIPY (registered trademark, manufactured by Invitrogen)-based dye molecules, Cy (registered trademark, manufactured by GE Healthcare)-based dye molecules, DY (registered trademark, manufactured by Dyomics GmbH)-based dye molecules, HiLyte (registered trademark, manufactured by AnaSpec Inc.)-based dye molecules, DyLight (registered trademark, manufactured by Thermo Fisher Scientific K.K.)-based dye molecules, ATTO (registered trademark, manufactured by ATTO-TEC GmbH)-based dye molecules, MFP (registered trademark, manufactured by Mobitec Co., Ltd.)-based dye molecules and the like can also be used. The generic names of these dye molecules are designated based on the main structure (skeleton) or registered trademark of the respective compounds; therefore, those of ordinary skill in the art should be able to properly understand the scope of fluorescent dyes belonging to the respective generic names without having to bear undue trial and error.

### • Fluorescent Substance-integrated Nanoparticle

"Fluorescent substance-integrated nanoparticles" are nano-sized particles having a structure in which particles made of an organic or inorganic substance is used as a matrix and plural fluorescent substances are encapsulated therein and/or adsorbed on the surface thereof. In this case, it is preferred that the matrix (e.g. resin) and the fluorescent substance (e.g. organic fluorescent dye) each comprise a substituent or a moiety having an opposite electric charge and that an electrostatic interaction occur therebetween.

Examples of the fluorescent substance to be contained in such fluorescent substance-integrated nanoparticles include, in addition to the above-described inorganic semiconductor nanoparticles and fluorescent dye molecules, "long-persistence phosphors" that comprise Y₂O₃, Zn₂SiO₄ or the like as a matrix and Mn²⁺, Eu³⁺ or the like as an activator.

Among matrices constituting the fluorescent substance-integrated nanoparticles, examples of the organic substance include resins that are generally classified into thermosetting resins, such as melamine resins, urea resins, aniline resins, guanamine resin, phenolic resins, xylene resins and furan resins; resins that are generally classified into thermoplastic resins, such as styrene resins, acrylic resins, acrylonitrile resins, AS resins (acrylonitrile-styrene copolymers) and ASA resins (acrylonitrile-styrene-methyl acrylate copolymers); other resins such as polylactic acids; and polysaccharides, and examples of the inorganic substance include silica (glass).

Fluorescent substance-integrated nanoparticles can be produced in accordance with a known method (see, for example, JP 2013-57937 A). More specifically, for example, fluorescent substance-containing silica particles in which silica is used as a matrix and a fluorescent substance is encapsulated therein can be produced by adding dropwise a solution, in which inorganic semiconductor nanoparticles, a fluorescent substance such as an organic fluorescent dye and a silica precursor such as tetraethoxysilane are dissolved, to a solution in which ethanol and ammonia are dissolved, and subsequently hydrolyzing silica precursor. Meanwhile, fluorescent substance-containing resin particles in which a resin is used as a matrix and a fluorescent substance is adsorbed to the surface of the resin particles or encapsulated in the resin particles can be produced by preparing in advance a solution of the resin or a dispersion of fine particles of the resin, adding thereto a fluorescent substance such as inorganic semiconductor nanoparticles and an organic fluorescent dye and then stirring the resultant.

Alternatively, fluorescent substance-containing resin particles can also be produced by adding a fluorescent dye to a solution of a resin material and then allowing polymerization reaction to proceed. For example, in cases where a thermosetting resin such as a melamine resin is used as a matrix resin, organic fluorescent dye-containing resin particles can be produced by: heating a reaction mixture, which contains a material of the resin (a monomer, an oligomer or a prepolymer, such as methylol melamine obtained by condensation of melamine and formaldehyde), an organic fluorescent dye, and preferably further a surfactant as well as a polymerization reaction accelerator (e.g. an acid) ; and thereby allowing polymerization reaction to proceed by an emulsion polymerization method. Further, in cases where a thermoplastic resin such as a styrene-based copolymer is used as a matrix resin, organic fluorescent dye-containing resin particles can be produced by: heating a reaction mixture, which contains a material of the resin, an organic fluorescent dye (as a resin material monomer, a monomer bound to an organic fluorescent dye through a covalent bond or the like in advance may also be used) and a polymerization initiator (e.g. benzoyl peroxide or azobis-isobutyronitrile); and thereby allowing polymerization reaction to proceed by a radical polymerization method or an ionic polymerization method.

The average particles size of the fluorescent substance-integrated nanoparticles (particularly, fluorescent dye-containing resin particles obtained by such a production method as described above) is not particularly restricted as long as it is suitable for immunostaining of a pathological specimen; however, taking into consideration the ease of detecting the fluorescent substance-integrated nanoparticles as bright spots, the average particle size is usually 10 to 500 nm, preferably 50 to 200 nm. Further, the variation coefficient, which represents the variation in the particle size, is usually 20% or less, preferably 5 to 15%. Fluorescent substance-integrated nanoparticles satisfying these conditions can be produced by adjusting the production conditions. For example, in the production of such fluorescent substance-integrated nanoparticles by an emulsion polymerization method, the particle size can be adjusted by the amount of a surfactant to be added and, generally speaking, when the amount of the surfactant is relatively large with respect to the amount of the parent materials of the fluorescent substance-integrated nanoparticles, the particle size tends to be small, whereas when the amount of the surfactant is relatively small, the particle size tends to be large.

The size of a fluorescent substance-integrated nanoparticle can be determined by taking an electron micrograph thereof using a scanning electron microscope (SEM), measuring the cross-sectional area of the fluorescent substance-integrated nanoparticle and then calculating the particle size as the diameter of a circle corresponding to the thus measured cross-sectional area with an assumption that the cross-sectional shape is circular. With regard to the average particle size and variation coefficient of a group of plural fluorescent substance-integrated nanoparticles, after determining the particle size for a sufficient number (for example, 1,000) of the fluorescent substance-integrated nanoparticles in the above-described manner, the average particle size is calculated as the arithmetic mean of the measured values and the variation coefficient is calculated by the following equation: 100 × (standard deviation of particle size)/(average particle size).

### • Reactive Moiety (Functional Group)

The label naturally comprises a reactive moiety that can bind to a divalent linking group or, otherwise, such a moiety is required to be introduced to the label after the production thereof.

For example, in cases where a fluorescent substance-integrated nanoparticle using a resin as its matrix is employed as the label, the particle-constituting resin itself originally comprises a reactive moiety or, otherwise, it is required to introduce a reactive moiety by surface modification after the particle formation. Specifically, for a melamine resin, a functional group such as an amino group can be utilized and, for an acrylic resin, a styrene resin or the like, by copolymerizing a monomer having a functional group (e.g. an epoxy group) in a side chain, the functional group itself or a functional group converted therefrom (e.g. an amino group generated by reaction with aqueous ammonia) can be utilized. In addition, by utilizing a reaction with such a functional group, other functional group(s) can also be introduced.

Further, in cases where a fluorescent substance-integrated nanoparticle using silica as its matrix is employed as the label, a desired functional group can be introduced by modifying the surface of the inorganic phosphor with a silane-coupling agent. For example, by using aminopropyltrimethoxysilane as the silane-coupling agent and allowing it to react with the above-described inorganic phosphor, an amino group can be introduced to the surface of the inorganic phosphor.

The above-described amino group contained in the label is capable of undergoing a reaction with a prescribed functional group, such as an NHS group, of a compound that serves as the origin of a divalent linking group and thereby forming a covalent bond. However, the reactive moiety of the label that can bind to a divalent linking group is not restricted to an amino group and, for example, a variety of known moieties (functional groups) that are reactive with a prescribed functional group, such as a carboxyl group, a thiol group, an NHS group or a maleimide group, can be utilized.

### (Divalent Linking Group)

The divalent linking group used in the present invention for linking the molecular target drug and the label can be selected from a variety of divalent linking groups that are used in the art, and the divalent linking group is not particularly restricted as long as the actions and effects of the present invention are exerted.

In the divalent linking group to be used in the present invention, one end thereof to be bound to the molecular target drug (the end different from the above-described group derived from NHS) is bound to a carbon atom of a nitrogen-containing aromatic ring. The method for forming such a divalent linking group is not particularly restricted, and a known reaction mode can be applied. Examples thereof include a mode in which, after allowing a compound (first reagent) which comprises a functional group that undergoes a reaction with a carbon atom of a nitrogen-containing aromatic ring to form a covalent bond to react with the molecular target drug, the resulting reaction product (intermediate) is subsequently allowed to react with a compound (second reagent) which comprises: a functional group that is reactive with other functional group different from the functional group of the first reagent; and a functional group for label binding, such as the above-described NHS-derived group.

As a reaction for allowing the divalent linking group to bind to a carbon atom of a nitrogen-containing aromatic ring, for example, the reaction described in Non-Patent Document 1 can be applied. In this reaction, a sodium salt of difluoroalkylazide group-containing sulfinic acid is used as a reaction reagent and the sulfinic acid group thereof undergoes a reaction with a carbon atom of a nitrogen-containing aromatic ring to form a covalent bond. Further, since an azide group reacts with an alkyne (carbon-carbon triple bond) to add a ring, a carbon-carbon triple bond-containing label or other reaction reagent having a carbon-carbon triple bond for constituting a part of the divalent linking group (the part that is not derived from the above-described reagent) can be bound.

It is preferred that, to the one end of the divalent linking group to bind to the label, a functional group (functional group for label binding) reactive with a functional group contained in the label be introduced in advance. Examples of such a functional group for label binding include a group that is derived from *N*-hydroxysuccinimide used as an activation reagent of carboxylic acids in organic chemistry and biochemistry (NHS group). *N*-hydroxysuccinimide undergoes dehydration-condensation with a carboxylic acid to form an unstable ester bond (active ester), and this active ester reacts with an amine to form an amide bond. Therefore, by utilizing an NHS group as a functional group for label binding, an amino group-containing label can be bound to one end of the divalent linking group.

Needless to say, the binding mode that can be utilized for linking the label to one end of the divalent linking group is not restricted to the above-described covalent bond formed by a reaction between an NHS group and an amino group, and a various kinds of binding modes can be utilized as long as the actions and effects of the present invention are exerted. For example, the molecular target drug and the label may be indirectly bound with each other (via the divalent linking group as well) by introducing biotin to one end of the divalent linking group while modifying the surface of the label with avidin (streptavidin) and subsequently allowing them to bind with each other through a biotin-avidin reaction.

### -Bioassay-

The bioassay of the present invention is performed using the labeling agent of the present invention, and the embodiment thereof is not particularly restricted. Examples thereof include a bioassay in which the labeling agent of the present invention is added to cultured cells and allowed to bind to a target molecule expressed in the cultured cells and the signal generated by the label is quantitatively obtained. By comparing the data of labeling agents using the molecular target drug (i.e. axitinib), the bioassay can be utilized for evaluating the strength of binding to the target molecule and improving the molecular target drug. Alternatively, the bioassay can also be utilized in an embodiment where the labeling agent of the present invention is added to a sample such as blood (serum) and allowed to bind to a specific molecule targeted by the molecular target drug or cells expressing the specific molecule and the molecule or cells are then qualitatively or quantitatively analyzed.

Such a bioassay can be modified as appropriate in accordance with "staining step" and "signal acquisition step" included in the histological staining method of the present invention as described below and the "staining step for morphological observation" that may be further included as required in the histological staining method of the present invention, such that the bioassay conforms to the use of cultured cells or a sample as a target in place of a tissue section. Moreover, depending on the purpose of the bioassay, when it is not necessary to obtain an image photographed by a digital camera, the bioassay can also be modified such that, for example, the fluorescence intensity is measured by a photomultiplier tube (photomultiplier, PMT).

### -Histological Staining Method-

The histological staining method of the present invention is performed using the labeling agent of the present invention, and the embodiment thereof is not particularly restricted; however, the histological staining method of the present invention comprises, for example, the following steps:
(1) the step of making a paraffin-embedded tissue section suitable for staining (pretreatment step);
(2) the step of staining a target molecule with the labeling agent (staining step);
(3) the step of making the thus stained tissue section suitable for observation (post-treatment step);
(4) as an optional step, the step of staining the tissue section such that the morphology of the cells and the like can be observed in a bright field (staining step for morphological observation); and
(5) the step of acquiring a signal generated by the label from the thus stained tissue section (signal acquisition step).

Specifically, these steps are performed by, for example, the below-described procedures.

### (Pretreatment Step)

Tissue sections are usually fixed with formalin and stored in a state of being embedded in paraffin. When such a tissue section is to be stained, in order to enable the staining, a pretreatment step is performed for deparaffinization and hydrophilization of the tissue section. This pretreatment step may also include, as required, a retrieval treatment for making proteins suitable for reaction with the molecular target drug.

The deparaffinization and hydrophilization can be performed by immersing the tissue section of interest in xylene to remove paraffin, subsequently immersing the tissue section in ethanol to remove xylene, and then further immersing the tissue section in water to remove ethanol. These three operations can be usually performed at room temperature. The immersion time in the respective operations may be 3 to 30 minutes or so and, as required, each treatment liquid may be replaced with a new one during the immersion.

The retrieval treatment is generally performed by immersing the tissue section in a retrieval liquid and heating the retrieval liquid. As the retrieval liquid, for example, 0.01 M citrate buffer (pH 6.0), 1 mM EDTA solution (pH 8.0), 5% urea or 0.1 M Tris-HCl buffer can be used. As a heating equipment, for example, an autoclave, a microwave oven, a pressure cooker or a water bath can be used. As for the temperature and time of the heating, for example, the heating can be performed at 50 to 130°C for 5 to 30 minutes.

### (Staining Step)

The staining step is a step of a staining treatment which allows the labeling agent to bind to the target molecule. The staining treatment can be performed by, for example, immersing the tissue section subjected to the pretreatment step into a solution containing the labeling agent (staining liquid). When immersing the tissue section in the staining liquid, the temperature, time and other conditions can be adjusted as appropriate in accordance with a conventional staining method such that an appropriate signal can be obtained.

In the staining step, in addition to the above-described treatment of staining the target molecule, a process of staining other biological substance (reference biological substance) may also be performed as required for the purposes of, for example, acquiring information for reference other than the binding between the target molecule and the molecular target drug used in the labeling agent. In that case, by immersing the tissue section subjected to the pretreatment step into a solution containing a labeling agent for staining the target molecule and a labeling agent for staining the reference biological substance, the two types of staining treatments can be performed at once.

### (Post-treatment Step)

After the completion of the staining step, the tissue section is preferably subjected to treatments such as fixation-dehydration, clearing and mounting such that the tissue section is made suitable for observation. The fixation-dehydration treatment can be performed by immersing the tissue section in a fixation liquid (a cross-linking agent such as formalin, paraformaldehyde, glutaraldehyde, acetone, ethanol or methanol) . The clearing can be performed by immersing the thus fixed and dehydrated tissue section in a clearing liquid (e.g. xylene). The mounting treatment can be performed by immersing the thus cleared tissue section in a mounting medium. The conditions for performing these treatments, such as the temperature and time of immersing the tissue section in each prescribed treatment liquid, can be adjusted as appropriate in accordance with a conventional staining method such that an appropriate signal can be obtained. By placing a cover glass on the tissue section after the post-treatment step, a specimen of a form suitable for morphological observation and signal acquisition is obtained.

### (Optional Step: Staining Step for Morphological Observation)

In the histological staining method using the labeling agent of the present invention, as required, a staining step for morphological observation can be incorporated for enabling to observe the morphology of cells, tissue, organ and the like in a bright field. The staining step for morphological observation can be performed in accordance with a conventional method. For the morphological observation of a tissue section, staining with eosin which stains cytoplasm, interstitial tissues, various fibers, erythrocytes and keratinocytes in red to dark red and/or staining with hematoxylin which stains cell nuclei, calcareous parts, cartilaginous tissues, bacteria and mucus in livid to light blue is/are typically performed, and a method of performing these two staining processes simultaneously is well known as "hematoxylin-eosin staining" (HE staining). When the staining step for morphological observation is incorporated, it may be performed after or before the above-described staining step.

### (Signal Acquisition Step)

The observation/photographing step is a step of acquiring a signal generated by the label from the tissue section subjected to the above-described staining step (and the staining step for morphological observation that is performed as required) . Since a phosphor is used as the label, the stained tissue section can be observed under a fluorescence microscope at a desired magnification while irradiating the tissue section with an excitation light appropriate for the phosphor, and a stained image on which the fluorescence intensity or the number of bright spots can be measured may then be photographed using a digital camera mounted on the fluorescence microscope. Particularly, since fluorescent substance-integrated nanoparticles are used as the phosphor, the fluorescent substance-integrated nanoparticles labeling the molecular target drug bound to the target molecule are easily observed as bright spots, so that not only the fluorescence intensity but also the number of bright spots can be measured.

In cases where the above-described treatment of staining a reference biological substance is performed in the staining step, a process of acquiring a signal generated by the label contained in the labeling agent used for staining the target molecule may also be performed in the signal acquisition step. Further, in cases where the above-described staining step for morphological observation is performed as an optional step, a process of observing the tissue section stained for morphological observation in a bright field and taking a photograph of the resulting stained image may also be performed before or after the signal acquisition step.

The thus photographed image can be used for quantifying the signal generated by the label in accordance with the purpose of the histological staining. Since a phosphor is used as the label, the intensity of fluorescence emitted by the phosphor and/or the number of fluorescent bright spots can be measured based on image processing.

Examples of a software that can be used in the image processing include "ImageJ" (open source). Utilization of such an image processing software enables to perform a process of extracting bright spots of a prescribed wavelength (color) from a stained image and determining their total brightness and a process of measuring the number of bright spots having a brightness of not less than a prescribed value in a semi-automatic and prompt manner.

Such quantitative data of the thus acquired signal can be applied in such a manner that, for example, by comparing the data of labeling agents using the molecular target drug, the strength of the binding to a target molecule is evaluated and utilized for improvement of the molecular target drug. Further, the quantitative data can also be utilized for estimating the effects of the molecular target drug and obtaining information useful for pathological diagnosis in such a manner that, for example, a target molecule contained in a tissue section (pathological specimen) derived from a patient of a disease targeted by the molecular target drug is quantified from the data of the tissue section and the molecular target drug is estimated to be highly effective when the target molecule is expressed in a large amount.

### EXAMPLES

[Labeling Agent Z] Axitinib-containing labeling agent in which a quantum dot (Qdot (registered trademark) 655) was bound to a carbon atom of the pyridine ring of axitinib via a divalent linking group

### (Synthesis Step Z-1)

An axitinib derivative (lb), in which a divalent linking group was bound to a carbon atom of the pyridine ring of axitinib (1), was synthesized by the following route. The thus obtained derivative was a mixture of regioisomers, and this mixture was used in the subsequent synthesis step. It is noted here that the route of the synthesis step Z-1 is based on the method described in Non-Patent Document 1.

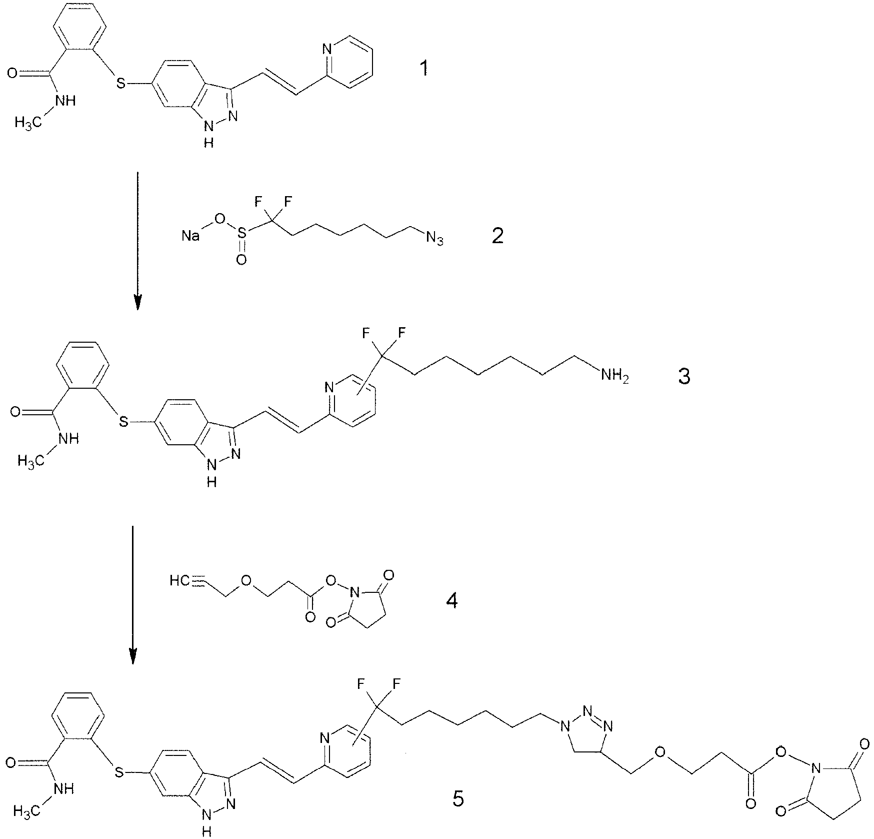

### (Synthesis Step Z-2)

By allowing the axitinib derivative (1) to react with quantum dots modified with PEG having an amino group at a terminal (Qdot 655 ITK Amino (PEG) Quantum Dots), a labeling agent Z in which Qdot was linked to a carbon atom of the pyridine ring of axitinib via a divalent linking group was obtained.

[Labeling Agent Y] Axitinib-containing labeling agent in which a quantum dot (Qdot (registered trademark) 655) was bound to the nitrogen atom of the amide group on the benzene ring of axitinib via a divalent linking group

### (Synthesis Step Y-1)

An axitinib derivative (6), in which a divalent linking group was bound to the nitrogen atom of the amide group (carbamoyl group) of axitinib, was synthesized referring to the sorafenib derivative-related method described in the paragraphs [0181] to [0189] of Patent Document 3 (Japanese Translated PCT Patent Application Laid-open No. 2012-533579). In the description of Patent Document 3, a sorafenib derivative in which a 2-hydroxyethylcarboxamide group was introduced to the pyridine ring is utlized to bind to a desired substrate (*ε*-polylysine); however, in this synthesis step Y-1, an axitinib derivative in which a 2-hydroxyethylcarboxamide group was introduced to the benzene ring was utilized.

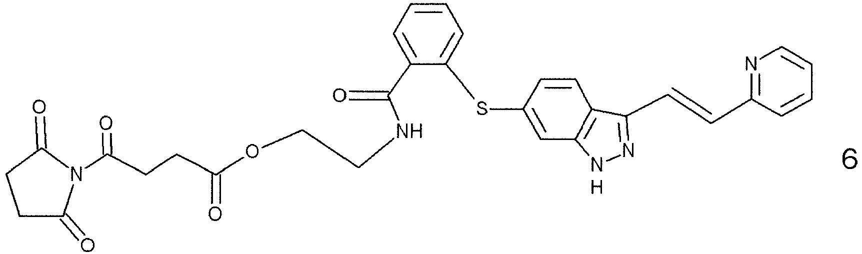

### (Synthesis Step Y-2)

By allowing the axitinib derivative (6) to react with quantum dots modified with PEG having an amino group at a terminal (Qdot 655 ITK Amino (PEG) Quantum Dots), a labeling agent Y in which Qdot was linked to the nitrogen atom of the amide group of axitinib via a divalent linking group was obtained.

### [Example 1]

A slide prepared by arranging spots of renal cell carcinoma tissue section on an array (USBiomax, Inc., T071) was subjected to deparaffinization and hydrophilization, and the labeling agent Z was added onto the tissue. After leaving the slide for 2 hours, unreacted labeling agent was removed by washing and a fluorescence image of the slide was taken under a confocal fluorescence microscope, followed by measurement of the fluorescence intensity.

In this step, a fluorescence microscope "BX-53" (manufactured by Olympus Corporation) was used for irradiation of excitation light and observation of emitted fluorescence, and a microscope digital camera "DP73" (manufactured by Olympus Corporation) mounted on this fluorescence microscope was used for photographing an immunostained image (×400). In conformity with Qdot 655 used as a phosphor, the wavelength of the irradiated excitation light was set at 415 to 455 nm using an optical filter for excitation light ("QD655-C", manufactured by OPTO-LINE, Inc.), and the wavelength of the fluorescence to be observed was set at 648 to 663 nm using an optical filter for fluorescence. The intensity of the excitation light used for the observation and image acquisition under the fluorescence microscope was set such that the irradiation energy was 30 W/cm² in the vicinity of the center of the visual field. In the image acquisition, the exposure time was adjusted to be 400 seconds such that the image brightness was not saturated.

Further, the measurement of the fluorescence intensity was performed by processing the photographed image using an image processing software "ImageJ" (open source). A region where the brightness of the fluorescence emitted by the phosphor is not less than a prescribed value was extracted, and the total intensity of the fluorescence constituting the region was determined.

### [Comparative Example 1]

The fluorescence intensity was measured in the same manner as in Example 1, except that the labeling agent Y was used in place of the labeling agent Z.

### [Results]

The results of Example 1 and Comparative Example 1 are shown in the table below. It is seen that the renal cell carcinoma tissue section yielded a stronger fluorescence intensity when stained with a labeling agent based on the binding mode of the present invention (labeling agent Z) than when stained with a labeling agent based on a conventional binding mode (labeling agent Y). From these results, it is speculated that, while the binding of axitinib conventionally used as a labeling agent to a target molecule (e.g. VEGFR) is easily disturbed by Qdot coupled as a phosphor, axitinib used in the labeling agent of the present invention is not likely to be disturbed by Qdot coupled as a phosphor and thus capable of strongly binding to a target molecule (e.g. VEGFR).

**[Table 1]**

| | Labeling agent | Fluorescence intensity |
|---|---|---|
| Example 1 | Labeling agent Z | 90 |
| Comparative Example 1 | Labeling agent Y | 7 |

## Claims

1. A labeling agent having a structure in which a molecular target drug, which is a compound comprising a nitrogen atom-containing aromatic ring, is bound to a label via a divalent linking group, wherein one end of said divalent linking group is bound to a carbon atom of said nitrogen atom-containing aromatic ring,
**characterized in that** the molecular target drug is axitinib and the label is a fluorescent substance-integrated nanoparticle.

2. The labeling agent according to claim 1, **characterized in that** said fluorescent substance-integrated nanoparticle comprises an organic fluorescent dye selected from the group consisting of a rhodamine-based dye, a squarylium-based dye, a cyanine-based dye, an aromatic ring-containing dye, an oxazine-based dye, a carbopyronine-based dye and a pyrromethene-based dye.

3. A bioassay, **characterized by** using the labeling agent according to claim 1 or 2.

4. A histological staining method, **characterized by** using the labeling agent according to claim 1 or 2.

## Patentansprüche

1. Markierungsmittel mit einer Struktur, in welcher ein molekulares Zielarzneimittel, bei dem es sich um eine einen stickstoffatomhaltigen aromatischen Ring umfassende Verbindung handelt, über eine zweiwertige Linkergruppe an eine Markierung gebunden ist, wobei ein Ende der zweiwertigen Linkergruppe an ein Kohlenstoffatom des stickstoffatomhaltigen aromatischen Rings gebunden ist,
**dadurch gekennzeichnet, dass** es sich bei dem molekularen Zielarzneimittel um Axitinib handelt und es sich bei der Markierung um einen Nanopartikel mit darin integrierter fluoreszierender Substanz handelt.

2. Markierungsmittel gemäß Anspruch 1, **dadurch gekennzeichnet, dass** der Nanopartikel mit darin integrierter fluoreszierender Substanz einen organischen Fluoreszenzfarbstoff ausgewählt aus der Gruppe bestehend aus einem Rhodamin-basierten Farbstoff, einem Squaryliumbasierten Farbstoff, einem Cyanin-basierten Farbstoff, einem einen aromatischen Ring enthaltenden Farbstoff, einem Oxazinbasierten Farbstoff, einem Carbopyronin-basierten Farbstoff und einem Pyrromethen-basierten Farbstoff umfasst.

3. Bioassay, **gekennzeichnet durch** Verwenden des Markierungsmittels gemäß Anspruch 1 oder 2.

4. Histologisches Färbeverfahren, **gekennzeichnet durch** Verwenden des Markierungsmittels gemäß Anspruch 1 oder 2.

## Revendications

1. Agent de marquage ayant une structure dans laquelle un médicament à cible moléculaire, qui est un composé comprenant un cycle aromatique contenant un atome d'azote, est lié à un marqueur par l'intermédiaire d'un groupe de liaison divalent, dans lequel une extrémité dudit groupe de liaison divalent est liée à un atome de carbone dudit cycle aromatique contenant un atome d'azote,
**caractérisé en ce que** le médicament à cible moléculaire est l'axitinib et le marqueur est une nanoparticule à substance fluorescente intégrée.

2. Agent de marquage selon la revendication 1, **caractérisé en ce que** ladite nanoparticule à substance fluorescente intégrée comprend un colorant fluorescent organique choisi dans le groupe constitué par un colorant à base de rhodamine, un colorant à base de squarylium, un colorant à base de cyanine, un colorant contenant un cycle aromatique, un colorant à base d'oxazine, un colorant à base de carbopyronine et un colorant à base de pyrrométhène.

3. Dosage biologique, **caractérisé par** l'utilisation de l'agent de marquage selon la revendication 1 ou 2.

4. Procédé de coloration histologique, **caractérisé par** l'utilisation de l'agent de marquage selon la revendication 1 ou 2.
